# EUROPEAN PATENT SPECIFICATION

(11) **EP 1 221 983 B1**
(45) Date of publication and mention of the grant of the patent: **03.12.2003**
(21) Application number: 00960900.9
(22) Date of filing: 20.09.2000
(51) Int. Cl.: A61L 2/10

(54) **A DEVICE FOR STERILIZING TAPS**
VORRICHTUNG ZUR STERILISATION VON WASSERHÄHNEN
DISPOSITIF DE STERILISATION DE ROBINETS

(30) Priority: 20.09.1999 IT TO990801
(43) Date of publication of application: 17.07.2002
(73) Proprietor: Magnino Prino, Renzo, 10050 Borgone di Susa (IT)
(72) Inventor: Magnino Prino, Renzo, 10050 Borgone di Susa (IT)
(74) Representative: Fioravanti, Corrado
(86) International application number: IB0001340
(87) International publication number: WO01021222

(56) References cited:
- WO-A-99/27970
- US-A- 5 738 780
- DATABASE WPI Section Ch, Week 199003 Derwent Publications Ltd., London, GB; Class D15, AN 1990-018185 XP002159276 & JP 01 297192 A (HOSHIN KAGAKU SANGY), 30 November 1989 (1989-11-30) cited in the application

## Description

The present invention relates to a device for sterilizing taps by irradiation with ultraviolet light.

As is known, any bacteria which are present in the environment and which are deposited on the end portions of delivery taps or pipes for transferring liquids and other substances which can be delivered by a tap may proliferate and cancel out the effect of the sterilizers normally provided upstream of the delivery devices.

The main object of the invention is to sterilize taps and not to permit bacterial reproduction or the propagation of bacteria upstream of the taps, particularly when the taps are used intermittently.

Japanese patent application JP-A-59136188 describes an ultraviolet lamp connected to a tap in order to sterilize liquids in the final portion of a pipe made of a material which transmits ultraviolet light. Similar devices are also described in JP-A-1297191, JP-A-1297192 and JP-A-1297193.

WO 99/27970 discloses a device for sterilizing taps by irradiation with ultraviolet light, comprising a diffuser element which is made of a material that transmits ultraviolet light, and which can be fitted to the end portion of the pipe of a tap and can be connected to a source of ultraviolet light by means of light guides in order to diffuse ultraviolet light onto the inner surface of the end portion through which the fluid delivered by the tap passes.

The object of the present invention is, in particular, to provide an improved device for sterilizing taps which is simpler, less expensive and more compact than that of the above-mentioned prior art.

A further object of the invention is to provide a device which can be fitted in a flexible manner on taps of any type.

These and other objects and advantages which will be understood better hereinafter, are achieved, according to the invention, by a device for sterilizing taps having the characteristics defined in the claims.

Some preferred but non-limiting embodiments of the invention will now be described with reference to the appended drawings, in which:
Figure 1 is a schematic view of a system for sterilizing taps according to the present invention,
Figure 2 is a schematic, vertically sectioned view of a tap provided with a sterilizing device that is not part of the present invention,
Figure 3 is a schematic view of a tap provided with a sterilizing device that is not part of the invention, and
Figure 4 is a schematic, perspective view of a covering element which can be fitted on the sterilizing device according to the invention.

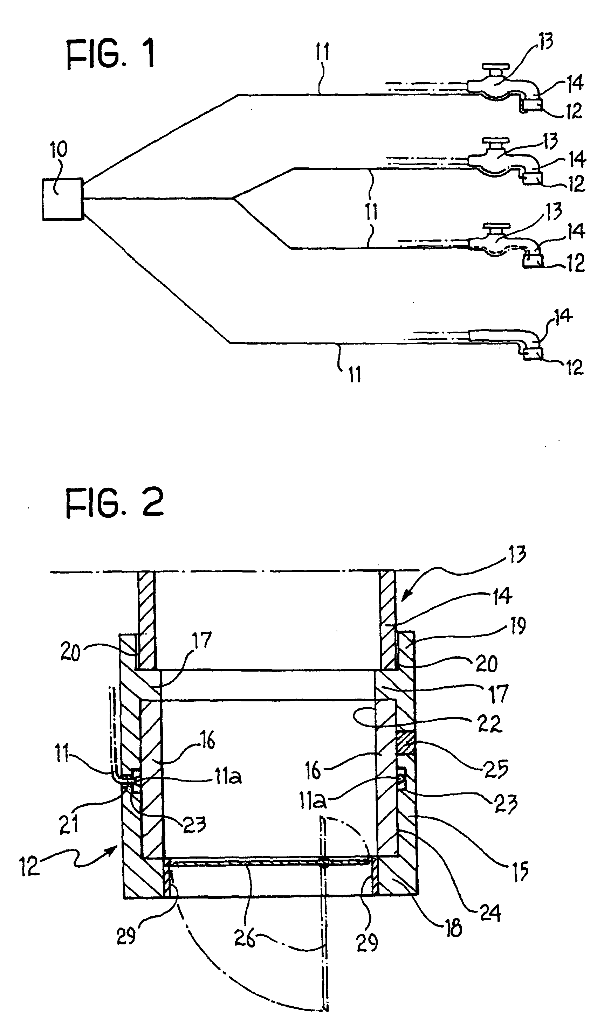

With reference initially to Figure 1, a source of ultraviolet light is schematically indicated 10; the light is transmitted, by means of light guides such as optical fibres 11, to a plurality of sterilizing devices 12, each fitted on a tap 13.

Neither the structural characteristics of the ultraviolet lamps nor those of the optical fibres (which may be of any known type, for example, of silica or synthetic) are relevant *per se* for the purposes of an understanding of the invention and they will therefore not be described in detail herein.

In the following description and in the claims, the expression "tap" will define both actual taps for delivering liquids such as, for example, drinking water and, more generally, the end portions of pipes for distributing a flow of liquid, semi-liquid, or granular substances, or powders, etc., which are to be delivered in conditions as free as possible from bacterial contamination.

With particular reference to Figure 2, a sterilizing device 12 is fitted on the end portion, indicated 14, of the pipe of a tap 13 and comprises, in this embodiment, a mounting sleeve 15 made of material which is opaque to ultraviolet light, in which a tubular diffuser element made of a material, for example, a vitreous material, which can transmit ultraviolet light is arranged coaxially.

The sleeve 15 has a pair of axially spaced-apart internal annular projections 17 and 18 which define a retaining seat for the diffuser 16. The upper end 19 of the sleeve 15 is configured for being coupled, preferably releasably, with the end portion 14 of the pipe of the tap, for example, by means of a threaded connection 20 or by means of a quick coupling (a snap or bayonet coupling, etc.) or by means of an external clamping band (not shown).

A hole 21 is formed in the sleeve 15 for the insertion of a cable or bundle of optical fibres 11 by means of which the ultraviolet light generated by the source 10 is transmitted, preferably continuously, and is diffused by the diffuser 16 onto the inner surface 22 of the end portion through which the fluid delivered by the tap passes.

To improve the diffusion of the light, the cable 11 may extend inside the sleeve 15, as in the embodiments described herein, forming a curved portion 11a which extends at least along a circumferential arc of greater or lesser extent around the diffuser 16, to which its end is connected, end on. In a preferred embodiment of the invention, the curved portion 11a is housed in a circular groove 23 formed in the inner wall 24 of the sleeve in the region of the hole 21.

The surface of the inside wall 24 is advantageously reflective to optimize the bactericidal action of the ultraviolet light on the internal walls of the device which are in contact with the flow delivered by the tap in operation.

In order to be sure than sterilization is taking place continuously before and during delivery, the sleeve 15 made of material opaque to the light has an indicator 25 indicative of the correct operative condition of the source 10 and/or of the cable 11. According to a particularly advantageous embodiment, the indicator 25 is constituted by a partially transparent window which selectively filters the ultraviolet light and allows only visible light which is not damaging to the human eye to shine through to the exterior.

According to an embodiment that is not part of the invention, a covering element 26 which can open automatically (as shown in broken outline) upon the arrival of the flow delivered may be mounted in the vicinity of the opening at the lower end of the sleeve. The covering element 26 is a disk which has dimensions matching those of the lower opening and which is mounted for pivoting on the sleeve so that, in the absence of a flow, it adopts a normally closed, horizontal condition, in which it does not allow the ultraviolet light to emerge from the opening, and in which, at the same time, it prevents bacteria entering the sleeve 15 and the pipe 14. The covering element 26 may be balanced so as to operate by gravity or may have a biasing spring (not shown) which tends to bring it to the horizontal closure position shown in continuous outline.

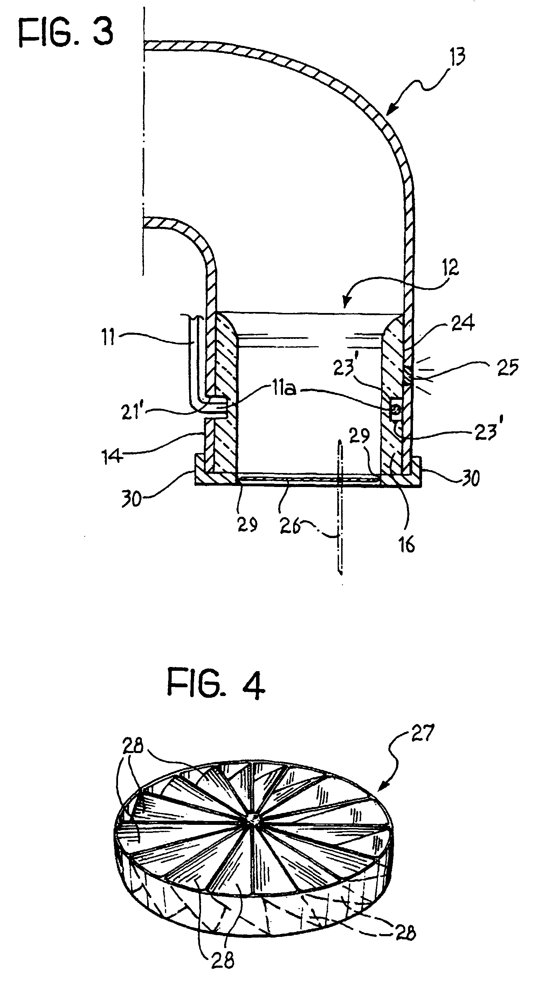

According to an embodiment according to the invention, a covering element 27 with an optical labyrinth (Figure 4) having an array of fixed vanes 28 inclined in the same direction and preventing light from emerging directly from inside the sleeve but allowing the flow to pass through, may be arranged on the lower end opening of the sleeve. The vanes 28 preferably have non-reflective surfaces (for example, satin-finished surfaces) for absorbing the ultraviolet radiation which strikes them.

The covering element 27, which may adopt, for example, a configuration including several pivoting portions, is coated with silver or with another material with similar antibacterial properties.

Again with reference to Figure 2, a surface layer of silver or of another substance with antibacterial properties is applied to the end portion 29 of the sleeve in the region of the opening for the outlet of the flow, to prevent proliferation of bacteria in the region downstream of the covering element 27 which is not reached by the ultraviolet light radiated by the diffuser 16.

According to another embodiment that is not part of the invention, shown in Figure 3, a diffuser element 16 similar to that already described with reference to the embodiment of Figure 2 is inserted or incorporated inside a pipe 14 of a tap in the vicinity of its outlet opening. The inner wall 24 of the pipe 14 is reflective in the region surrounding the diffuser 16.

The diffuser 16, which preferably has a diameter matching the inside diameter of the pipe 14, is held in position by a retaining element 30 in the form of a flanged, possibly threaded, ring. According to the invention the retaining element 30, which is preferably silvered, carry a labyrinth covering element 27 of the type illustrated separately in Figure 4 instead of a pivoting covering element 26 as shown in Fig.3.

The optical-fibre cable 11 extends through a hole 21' formed directly in the pipe 14 and is housed in a groove 23' formed in the diffuser 16.

According to a further variant, not illustrated, the retaining element 30 may be formed integrally with the tap.

Those skilled in the art will understand that it is possible to effect many other variations to the embodiments described herein without departing from the scope of the claims, particularly, in order to bring the cable or light guide 11 into the region in which the ultraviolet light is to be diffused. For example, the cable could run along the outside or the inside of the pipe 14 or could even be incorporated in the body of the tap.

As will be appreciated, the invention enables a plurality of taps to be served by a single source of ultraviolet light, thus providing a system for sterilizing taps. The invention, which can easily be applied to pre-existing taps, can be used advantageously in homes, in public places such as hospitals, restaurants, etc., and in industry, particularly in the food industry.

## Claims

1. A device for sterilizing taps by irradiation with ultraviolet light, comprising:
- a diffuser element (16) which is made of a material that transmits ultraviolet light, and which can be fitted to the end portion of the pipe (14) of a tap (13) and can be connected to a source of ultraviolet light (10) by means of light guides (11) in order to diffuse ultraviolet light onto the inner surface of the end portion through which the fluid delivered by the tap (13) passes;
**characterised by** further comprising:
- a covering element (26, 27) mounted downstream of the diffuser element (16), the covering element (26, 27) comprising an optical labyrinth including several labyrinth surfaces (28) allowing the flow to be delivered while preventing ultraviolet light from emerging directly from the opening for the outlet of the flow delivered.

2. A sterilizing device according to Claim 1, wherein the diffuser element (16) is housed inside a sleeve (15) made of material which is opaque to ultraviolet light and which can be mounted on the end portion (14) of the pipe of a tap (13).

3. A sterilizing device according to Claim 1, wherein the diffuser element (16) is of a tubular, cylindrical shape.

4. A sterilizing device according to Claim 1, wherein the diffuser element (16) is housed inside a wall with a reflective surface (24).

5. A sterilizing device according to Claim 1, comprising visible means (25) indicative of the operating condition of the device (12).

6. A sterilizing device according to Claim 5, in which the visible means comprise a partially transparent window (25) formed in a wall (14, 15) adjacent the diffuser element (16).

7. A sterilizing device according to Claim 1, wherein the surfaces (26, 27, 28, 29, 30) situated downstream of the diffuser element (16) comprise a material with antibacterial properties.

8. A sterilizing device according to Claim 1, wherein the diffuser element (16) can be fitted inside a pipe (14) of a tap (13) in the vicinity of the outlet opening thereof.

9. A sterilizing device according to Claim 8, comprising a retaining means (30) for holding the diffuser element (16) in the pipe (14).

10. A sterilizing device according to Claim 1, wherein the optical labyrinth includes an array of fixed vanes (28) inclined in the same direction.

11. A sterilizing device according to Claim 1, wherein the covering element includes pivoting portions.

12. A system for sterilizing a plurality of taps (13, 14) each provided with a sterilizing device (12) according to Claim 1, in which at least two devices (12) of two different taps are connected to a same source (10) of ultraviolet light by optical-fibre transmission means (11).

## Patentansprüche

1. Vorrichtung zum Sterilisieren von Hähnen durch eine Bestrahlung mit ultraviolettem Licht, wobei die Vorrichtung enthält:
- ein Streuelement (16), das aus einem Material besteht, das ultraviolettes Licht überträgt, und das am Endteil des Rohrs (14) eines Hahns (13) angebracht und mit einer Quelle von ultraviolettem Licht (10) über Lichtleiter (11) verbunden werden kann, um das ultraviolette Licht auf die Innenfläche des Endteils zu streuen, durch den das vom Hahn (13) gelieferte Fluid strömt;
**dadurch gekennzeichnet, dass** die Vorrichtung weiters enthält:
- ein Verschlusselement (26, 27), das stromabwärts des Streuelements (16) angebracht ist, wobei das Verschlusselement (26, 27) ein optisches Labyrinth enthält, das mehrere Labyrinthflächen (28) aufweist, die eine Abgabe der Strömung ermöglichen, während verhindert wird, dass ultraviolettes Licht direkt aus jener Öffnung austritt, die den Auslass für die abgegebene Strömung bildet.

2. Sterilisiervorrichtung gemäß Anspruch 1, wobei das Streuelement (16) innerhalb einer Buchse (15) untergebracht wird, die aus einem Material besteht, das für ultraviolettes Licht undurchlässig ist, und die am Endteil (14) des Rohrs eines Hahns (13) befestigt werden kann.

3. Sterilisiervorrichtung gemäß Anspruch 1, wobei das Streuelement (16) die Form eines zylindrischen Rohres besitzt.

4. Sterilisiervorrichtung gemäß Anspruch 1, wobei das Streuelement (16) innerhalb einer Wand mit einer reflektierenden Oberfläche (24) untergebracht ist.

5. Sterilisiervorrichtung gemäß Anspruch 1, wobei die Vorrichtung eine optische Einrichtung (25) enthält, die den Betriebszustand der Vorrichtung (12) anzeigt.

6. Sterilisiervorrichtung gemäß Anspruch 5, wobei die optische Einrichtung ein teilweise durchsichtiges Fenster (25) enthält, das in einer Wand (14, 15) neben dem Streuelement (16) ausgebildet ist.

7. Sterilisiervorrichtung gemäß Anspruch 1, wobei die Flächen (26, 27, 28, 29, 30), die stromabwärts des Streuelements (16) angeordnet sind, ein Material enthalten, das über antibakterielle Eigenschaften verfügt.

8. Sterilisiervorrichtung gemäß Anspruch 1, wobei das Streuelement (16) in ein Rohr (14) eines Hahns (13) in der Nähe von dessen Auslassöffnung eingesetzt werden kann.

9. Sterilisiervorrichtung gemäß Anspruch 8, wobei die Vorrichtung eine Einspanneinrichtung (30) enthält, um das Streuelement (16) im Rohr (14) zu halten.

10. Sterilisiervorrichtung gemäß Anspruch 1, wobei das optische Labyrinth eine Anordnung von ortsfesten Flügeln (28) aufweist, die in die selbe Richtung geneigt sind.

11. Sterilisiervorrichtung gemäß Anspruch 1, wobei das Verschlusselement verschwenkbare Teile aufweist.

12. System zum Sterilisieren einer Vielzahl von Hähnen (13, 14), von denen jeder mit einer Sterilisiervorrichtung (12) gemäß Anspruch 1 ausgestattet ist, wobei zumindest zwei Vorrichtungen (12) von zwei verschiedenen Hähnen über Übertragungseinrichtungen (11) aus optischen Fasern mit der selben Quelle (10) von ultraviolettem Licht verbunden sind.

## Revendications

1. Dispositif de stérilisation de robinets par irradiation avec un rayonnement ultraviolet, comprenant :
un élément formant diffuseur (16) qui est composé d'un matériau qui transmet un rayonnement ultraviolet et qui peut être monté à la partie d'extrémité du tuyau (14) d'un robinet (13) et peut être relié à une source de rayonnement ultraviolet (10) au moyen de guides de rayonnement (11) afin de diffuser le rayonnement ultraviolet sur la surface intérieure de la partie d'extrémité à travers laquelle le liquide fourni par le robinet (13) passe ;
**caractérisé en ce qu'**il comprend en outre :
un élément formant couvercle (26, 27) monté en aval de l'élément formant diffuseur (16), l'élément formant couvercle (26, 27) comprenant un labyrinthe optique comprenant plusieurs surfaces de labyrinthe (28) permettant à l'écoulement d'être fourni tout en empêchant le rayonnement ultraviolet d'émerger directement de l'ouverture pour l'orifice de sortie de l'écoulement fourni.

2. Dispositif de stérilisation selon la revendication 1, dans lequel l'élément formant diffuseur (16) est logé à l'intérieur d'un manchon (15) composé de matériau qui est opaque au rayonnement ultraviolet et qui peut être monté sur la partie d'extrémité (14) du tuyau d'un robinet (13).

3. Dispositif de stérilisation selon la revendication 1, dans lequel l'élément formant diffuseur (16) est de forme cylindrique, tubulaire.

4. Dispositif de stérilisation selon la revendication 1, dans lequel l'élément formant diffuseur (16) est logé à l'intérieur d'une paroi avec une surface réfléchissante (24).

5. Dispositif de stérilisation selon la revendication 1, comprenant des moyens visibles (25) indiquant l'état de fonctionnement du dispositif (12).

6. Dispositif de stérilisation selon la revendication 5, dans lequel les moyens visibles comprennent une fenêtre partiellement transparente (25) formée dans une paroi (14, 15) adjacente à l'élément formant diffuseur (16).

7. Dispositif de stérilisation selon la revendication 1, dans lequel les surfaces (26, 27, 28, 29, 30) situées en aval de l'élément formant diffuseur (16) comprennent un matériau avec des propriétés antibactériennes.

8. Dispositif de stérilisation selon la revendication 1, dans lequel l'élément formant diffuseur (16) peut être monté à l'intérieur d'un tuyau (14) d'un robinet (13) à proximité de l'ouverture de l'orifice de sortie de celui-ci.

9. Dispositif de stérilisation selon la revendication 8, comprenant des moyens de retenue (30) destinés à maintenir l'élément formant diffuseur (16) dans le tuyau (14).

10. Dispositif de stérilisation selon la revendication 1, dans lequel le labyrinthe optique comprend une série de valeurs fixes (28) inclinées dans la même direction.

11. Dispositif de stérilisation selon la revendication 1, dans lequel l'élément formant couvercle comprend des parties pivotantes.

12. Système de stérilisation d'une pluralité de robinets (13, 14), chacun proposé avec un dispositif de stérilisation (12) selon la revendication 1, dans lequel au moins deux dispositifs (12) de deux robinets différents sont reliés à la même source (10) de rayonnement ultraviolet par des moyens de transmission par fibre optique (11).
